(19) **Europäisches Patentamt / European Patent Office / Office européen des brevets**

(11) **EP 2 861 278 B1**

(12) **FASCICULE DE BREVET EUROPEEN**

(45) Date de publication et mention de la délivrance du brevet:
**25.10.2017 Bulletin 2017/43**

(21) Numéro de dépôt: **13730517.3**

(22) Date de dépôt: **13.06.2013**

(51) Int Cl.:
*A61M 5/168* (2006.01)  *A61M 5/172* (2006.01)
*A61M 5/148* (2006.01)  *A61M 5/142* (2006.01)

(86) Numéro de dépôt international:
**PCT/EP2013/062215**

(87) Numéro de publication internationale:
**WO 2013/186290 (19.12.2013 Gazette 2013/51)**

(54) **CONTROLEUR POUR L'ASSERVISSEMENT D'UN DISPOSITIF D'INJECTION**

STEUERUNG ZUM AUTOMATISCHEN STEUERN EINER VORRICHTUNG

CONTROLLER FOR THE AUTOMATIC CONTROL OF AN INJECTION DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.06.2012 FR 1255531**
**13.06.2012 US 201261659330 P**

(43) Date de publication de la demande:
**22.04.2015 Bulletin 2015/17**

(73) Titulaire: **Medex**
**69800 Saint-Priest (FR)**

(72) Inventeurs:
• **REBERGUE, Habib**
**F-69008 Lyon (FR)**

• **MURILLO, Adriana**
**F-69500 Bron (FR)**
• **DGHOUGHI, Ali**
**F-38000 Grenoble (FR)**

(74) Mandataire: **Regimbeau**
**87 rue de Sèze**
**69477 Lyon Cedex 06 (FR)**

(56) Documents cités:
EP-A1- 1 788 498      WO-A1-2008/099876
WO-A1-2012/176170      KR-A- 20020 036 248
KR-A- 20110 123 081      US-A1- 2011 318 198

**Description**

## DOMAINE TECHNIQUE

La présente invention concerne le domaine technique général des dispositifs d'injection de produit de contraste.

## PRESENTATION GENERALE DE L'ART ANTERIEUR

Les développements de la médecine ont conduit à mettre au point différents procédés d'analyse et de contrôle de l'état des patients. Parmi ces procédés, il existe les analyses faites après injection d'un produit de contraste, par exemple pour l'imagerie médicale qui regroupe aussi bien l'imagerie par rayons X que l'imagerie par Résonance Magnétique (IRM) ou la médecine nucléaire.

On connaît des dispositifs pour injecter un produit liquide tel qu'un produit de contraste à un patient comme par exemple les techniques de pompe en ligne, les techniques dites « *pousse-seringue »,* ou encore les techniques d'injecteur à poche.

Dans le cadre d'une injection, l'utilisateur saisit sur un clavier un protocole d'injection en renseignant notamment le débit d'injection désiré en sortie du dispositif d'injection dit « *débit de consigne ».*

Une difficulté liée à l'injection de produit liquide à l'aide d'un tel dispositif concerne le maintien d'un débit de sortie conforme au débit de consigne.

Le document WO 97/45150 propose d'asservir un dispositif d'injection en fonction d'un débit mesuré en sortie du dispositif d'injection. Le dispositif d'injection comprend des moyens d'entrainement pour l'injection du produit liquide, un débitmètre disposé en sortie du dispositif d'injection et une unité de contrôle générant une commande moteur pour contrôler l'actionnement électrique des moyens d'entrainement. L'unité de contrôle est reliée au débitmètre pour recevoir un signal représentatif de la vitesse d'écoulement du produit liquide sortant du dispositif d'injection. L'unité de contrôle fait varier la commande moteur en fonction de ce signal.

Un inconvénient de ce dispositif concerne d'une part le coût des consommables qui comportent un système de débitmètre jetable, et d'autre part la faible qualité de la mesure de débit réalisée par le débitmètre qui présente une précision trop faible. Le document KR 2002 0036248 A divulgue les caractéristiques du préambule des revendications 1 et 9. Un but de l'invention est de proposer un dispositif d'injection de produit liquide permettant de diminuer le coût des consommables et d'améliorer la précision dans l'évaluation du débit de sortie de l'injecteur.

## PRESENTATION DE L'INVENTION

A cet effet, l'invention propose un système d'injection à un patient d'un produit liquide contenu dans un récipient, le système comprenant une unité de mise en pression pour l'entrainement du produit liquide contenu dans le récipient, remarquable en ce que le système comprend en outre un calculateur programmé pour mettre en oeuvre les étapes suivantes :

- Recevoir une information représentative d'un débit réel d'avancement de l'unité de mise en pression,
- Recevoir une information représentative d'une pression à l'intérieur du récipient,
- Estimer un débit de fuite fictif en fonction d'une loi de déformation du système d'injection sous l'effet de la pression à l'intérieur du récipient,
- Estimer en continu un débit de sortie du récipient correspondant à la différence entre le débit réel d'avancement de l'unité de mise en pression et le débit de fuite fictif.

Par « information représentative », on entend désigner un signal obtenu d'un détecteur, par exemple un codeur de position ou un capteur de pression. Ce signal peut par exemple être obtenu par la mesure d'une force via une jauge de contrainte ou via un courant. Il peut y avoir un rapport de conversion appliqué entre la valeur obtenue à travers ce signal et la valeur réellement transmise au calculateur.

On entend dans le cadre de la présente invention par « estimation », le calcul du débit d'injection à partir d'une ou plusieurs valeurs instantanées acquises. Par "estimation en continu", on entend la répétition de ce calcul du débit d'injection à intervalles réguliers pendant une certaine durée qui peut être égale à la durée d'injection.

On entend par « débit réel d'avancement », le débit généré par l'unité de mise en pression en fonction d'un débit de consigne saisi par l'utilisateur.

On entend par « fuite fictive » dans une conduite, l'ensemble des phénomènes physiques influant sur un débit de sortie, à l'exception des déperditions de liquide qui sont considérées comme une fuite réelle. Ces phénomènes peuvent comprendre par exemple la compressibilité du fluide, la déformation de la conduite ou encore le refoulement du fluide dans la conduite.

On entend par « débit de fuite fictif », une quantité de liquide par unité de temps égale à un écart entre un débit imposé par un injecteur et un débit éjecté hors d'un récipient contenant le produit liquide à injecter. Ce débit est qualifié de « débit de fuite fictif » en ce qu'il n'y a pas, dans le système d'injection, de déperdition de liquide entre le récipient contenant le produit liquide à injecter et la sortie du système d'injection.

Le débit de fuite fictif correspond donc :

- pendant la phase d'injection (phase durant laquelle l'unité de mise en pression exerce une force sur le récipient pour éjecter le produit liquide du récipient), à une quantité de liquide par unité de temps qui n'est pas éjectée du récipient,
- pendant la phase d'arrêt (phase durant laquelle l'unité de mise en pression n'exerce pas de force sur le récipient), à une quantité de liquide par unité de temps qui s'écoule du récipient (écoulement résiduel hors du récipient en l'absence de force exercée par l'unité de mise en pression sur le récipient).

Ainsi et comme décrit ci-dessus, l'invention propose le calcul d'un débit de fuite fictif à partir d'une loi de déformation du système d'injection en fonction de la pression à l'intérieur du récipient.

Le lecteur appréciera que cette déformation du système est du type élastique (i.e. que cette déformation est réversible), par opposition à une déformation plastique ou à une usure (i.e. une déformation non réversible). Par ailleurs, le lecteur appréciera que, dans le cadre de la présente invention, on entend par « système », l'ensemble composé :

- de l'unité de mise en pression et
- du récipient,

cet ensemble ne comprenant (i.e. étant dépourvu de) :

- ni la connectique d'amenée de liquide au patient,
- ni le produit de contraste en tant que tel.

Ainsi, la loi de déformation du système tient compte de la déformation de l'unité de mise en pression et de la déformation du récipient.

Des aspects préférés mais non limitatifs du système selon l'invention sont les suivants :

- le calculateur estime le débit de fuite fictif en fonction de la pression à l'intérieur du récipient,
- l'unité de mise en pression est constituée d'au moins une enceinte destinée à recevoir une poche contenant le produit liquide à injecter et comprenant au moins un orifice d'écoulement destiné à être connecté à une tubulure reliée au patient par l'intermédiaire d'un conduit d'injection, l'enceinte comportant au moins une membrane déformable à volume variable sous l'action d'un fluide moteur injecté dans ladite enceinte de sorte à comprimer la poche afin de forcer le produit liquide à injecter à s'écouler dans la tubulure,
- le calculateur inclut un régulateur auto-ajustable - tel qu'un régulateur de type proportionnel intégral - le calculateur ajustant automatiquement des paramètres du régulateur - tels que des coefficients Ki, Kp - en fonction de la pression à l'intérieur du récipient et du débit de consigne saisi par l'utilisateur,
- le calculateur est programmé pour déterminer un débit de sortie de référence en fonction d'une information relative à la nature du produit liquide contenu dans le récipient,
- le calculateur est programmé pour :

  - comparer le débit de sortie de référence au débit de sortie estimé et
  - émettre une alarme si la différence entre le débit de sortie de référence et le débit de sortie estimé est supérieure à une valeur seuil,

- le système comprend en outre un afficheur pour l'affichage d'un graphique représentant un débit de sortie du récipient en fonction du temps, le débit de sortie étant égal à la différence entre le débit de consigne et le débit de fuite fictif estimé,
- le produit liquide est un produit de contraste.

L'invention concerne également un procédé de contrôle d'un système d'injection à un patient d'un produit liquide contenu dans un récipient, ayant les caractéristiques de la revendication 9.

## PRESENTATION DES FIGURES

D'autres caractéristiques et avantages de l'invention ressortiront encore de la description qui suit, laquelle est purement illustrative et non limitative et doit être lue en regard des dessins annexés, sur lesquels :

- les figures 1 et 2 illustrent une variante de réalisation d'un dispositif d'injection,
- les figures 3 et 4 illustrent deux exemples de poche contenant un produit liquide injectable,
- les figures 5 et 6 illustrent les positions fermée et ouverte d'un mode de réalisation d'une unité de mise en pression,
- la figure 7 illustre une deuxième variante de réalisation d'un dispositif d'injection,
- la figure 8 illustre un exemple de demi-coque du dispositif illustré à la figure 7,
- les figures 9 et 10 illustrent une vessie du dispositif illustré à la figure 7,
- les figures 11 et 12 illustrent un coussin du dispositif illustré à ta figure 7,
- la figure 13 illustre une loi de déformation,
- la figure 14 illustre des fonctions proportionnelle et intégrale.
- la figure 15 illustre un algorithme d'estimation du débit de sortie du dispositif illustré à la figure 7.

## DESCRIPTION DE MODES DE REALISATION DE L'INVENTION

On va maintenant décrire plus en détail l'invention en référence aux figures. Dans ces différentes figures, les éléments équivalents portent les mômes références numériques.

### 1. Estimation du débit de sortie du dispositif d'injection

Au lieu de mesurer directement le débit en sortie du dispositif d'injection, l'invention propose d'estimer ce débit en sortie du dispositif d'injection.

Ceci permet d'une part d'améliorer la précision dans l'évaluation du débit de sortie de l'injecteur. En effet, la plupart des débitmètres ne tiennent pas compte des variations possibles dans la masse volumique du produit liquide contenu dans le récipient, ce qui est une source d'erreur dans la mesure du débit de sortie.

Ceci permet d'autre part de diminuer les coûts des consommables, les débitmètres utilisés pour réaliser des mesures de débit en sortie d'un dispositif d'injection étant généralement jetables.

### 1.1. Dispositif de type pousse seringue

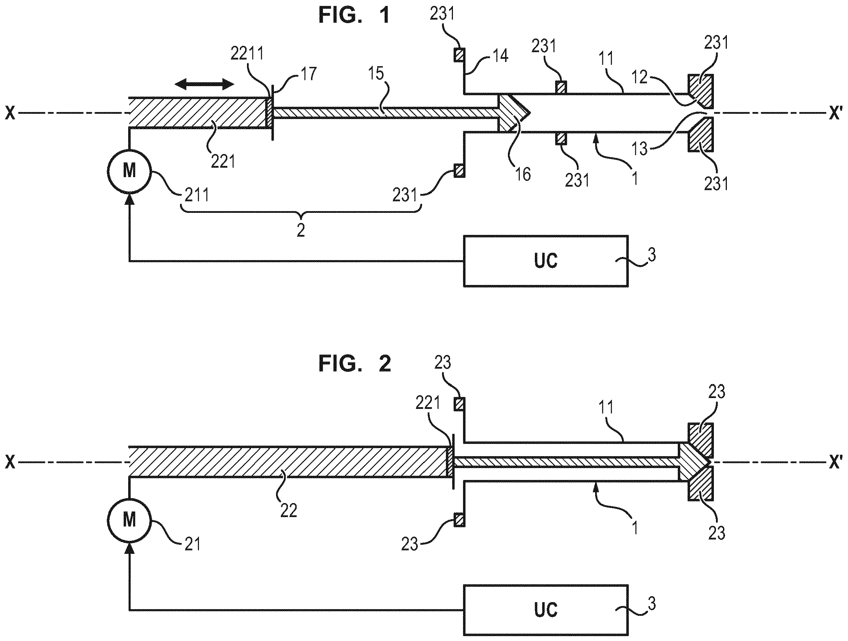

En référence aux figures 1 et 2, on a illustré un exemple de dispositif d'injection de type pousse-seringue. Le dispositif comprend une unité de mise en pression 2, une unité de contrôle 3.

Le dispositif d'injection permet l'injection d'un produit liquide contenu dans une seringue 1.

#### 1.1.1. Seringue

La seringue 1 comprend un corps cylindrique 11 dont la partie avant 12 converge jusqu'à un conduit de sortie 13 équipé d'un raccord pour un tube souple (non représenté). L'extrémité arrière du corps cylindrique 11 est pourvue d'une collerette extérieure radiale 14, de forme extérieure sensiblement circulaire.

On entend, dans le cadre de la présente invention, par « forme extérieure sensiblement circulaire » une forme circulaire tronquée ou une forme ovale,

Dans le corps cylindrique 11 est disposé un piston 15. La face avant de ce piston est recouverte d'un joint élastomère 16 et a une forme conique conjuguée de la partie avant 12 du corps cylindrique 11 de la seringue 1. Le joint 16 se prolonge vers l'arrière de manière à provoquer un frottement avec la paroi intérieure du corps cylindrique 11. La face arrière du piston 15 est plane et munie en son centre d'un pion 17 en forme de champignon à section circulaire.

#### 1.1.2. Unité de mise en pression

L'unité de mise en pression 2 comprend des moyens d'entrainement composés :

- d'un moteur 211,
- d'un support 231 pour maintenir la seringue en position fixe, et
- d'un poussoir 221 mobile en translation suivant un axe X-X' sous la commande de l'unité de contrôle 3.

Le moteur 211 permet le déplacement du poussoir 221 selon l'axe X-X'.

Le support 231 est constitué de demi-disques disposés le long du corps cylindrique 11 de la seringue 1 et prolongé vers l'avant par un berceau semi-cylindrique destiné à recevoir la partie avant du corps cylindrique. La forme du berceau est complémentaire de la forme conique de la partie avant du corps cylindrique 11. Le berceau comprend un évidement pour le passage du conduit de sortie 13 du corps cylindrique 11.

Le poussoir 221 est de forme générale cylindrique. Il comporte une tête avant 2211 destinée à venir en contact avec le pion 17 du piston 15 de la seringue 1. Le poussoir 221 peut être déplacé entre une position déployée (cf. figure 1) et une position rétractée (cf. figure 2), la tête avant 2211 du poussoir 221 étant plus proche du berceau semi-cylindrique du support 231 dans la position rétractée que dans la position déployée.

### 1.1.3. Unité de contrôle

L'unité de contrôle 3 permet de commander l'unité de mise en pression.

Plus précisément, l'unité de contrôle est destinée à générer un signal électrique de commande au moteur de l'unité de mise en pression, en fonction notamment d'un débit de consigne saisi par l'utilisateur du dispositif d'injection sur des moyens de saisie - tel qu'un clavier, un écran tactile, etc. - du dispositif d'injection.

L'unité de contrôle peut comprendre un calculateur permettant notamment d'estimer le débit en sortie de l'unité de mise en pression comme il sera décrit plus en détail dans la suite.

Le calculateur est par exemple un/des ordinateur(s), un/des processeur(s), un/des microcontrôleur(s), un/des micro-ordinateur(s), un/des automate(s) programmable(s), un/des circuit(s) intégré(s) spécifique(s) d'application, d'autres circuits programmables, ou d'autres dispositifs qui incluent un ordinateur tels qu'une station de travail.

L'unité de contrôle peut être intégrée à l'unité de mise en pression, ou être séparée de l'unité de mise en pression.

Le calculateur est couplée à une (ou plusieurs) mémoire(s) qui peut être intégrée au ou séparée du calculateur. La mémoire peut être une mémoire ROM/RAM, une clé USB, une mémoire d'un serveur central. Cette mémoire peut permettre de stocker les débits estimés par le calculateur, ou d'autres données utilisées par le calculateur.

### 1.1.4. Exemple de méthode d'estimation du débit de sortie par le calculateur

### 1.1.4.1. Mesure de la pression

Pour estimer le débit en sortie du dispositif d'injection, le calculateur peut utiliser comme information une mesure de la pression à l'intérieur de la seringue. Cette mesure de pression peut être obtenue de différentes manières.

Par exemple, il peut être prévu un capteur de pression à l'intérieur de la seringue.

En variante, le dispositif d'injection peut comprendre une jauge de contrainte 2212 pour mesurer la force appliquée par l'unité de mise en pression sur le piston de la seringue. Cette jauge de contrainte 2212 peut être positionnée entre la tête avant 2211 du poussoir 221 et le pion 17 du piston 15 de la seringue 1. Connaissant la surface S de la face avant du piston 15 et la force F exercée par le poussoir 221 sur le piston 15 étant perpendiculaire à cette surface S, on obtient la pression p par la formule suivante :

$$p = \frac{F}{S}$$

Avec :

- p la pression à l'intérieur de la seringue (donnée déduite par le calculateur),
- F la force appliquée par le poussoir sur le piston de la seringue (mesure issue de la jauge de contrainte), et
- S la surface de la face avant du piston (de valeur constante : la taille de la seringue étant déterminée par le berceau).

### 1.1.4.2. Principe de la Loi de déformation

Avantageusement, le calculateur peut également utiliser une loi de déformation du dispositif pour l'estimation du débit de sortie du dispositif d'injection.

Une loi de déformation vise à modéliser notamment le comportement des solides par des lois empiriques lors de leur déformation.

Cette loi de déformation permet de prendre en compte un débit de fuite fictif dans le calcul du débit de sortie du dispositif d'injection. Le principe du débit de fuite peut être expliqué comme suit.

Le débit d'injection qui est affiché sur certains dispositifs d'injection existants n'est pas une valeur mesurée ni une estimation du débit de sortie de l'injecteur. Ce débit affiché est basé sur la vitesse d'avancement du poussoir. A l'état stable (i.e. régime nominal ou établi), l'indication correspond au débit réel. Par contre lors des états transitoires, le débit instantané en sortie de l'injecteur reste inconnu.

En effet, les écoulements ne sont pas parfaits : de nombreux frottements opèrent dans les tubulures. Ces frottements sont fonction de la viscosité du produit liquide injecté, du débit d'injection, des caractéristiques des tubulures (rugosité, diamètre intérieur,...) et des cathéters. Ces frottements, appelés pertes de charge, se traduisent par une montée en pression à l'intérieur du récipient contenant le produit liquide à injecter. Le dispositif d'injection se déforme sous l'effet de cette pression. Pendant le temps de la montée en pression (régime transitoire), une partie du volume déplacé par le piston est « perdu » dans le contenant ainsi déformé (i.e. n'est pas expulsé vers la sortie du piston). Un écart entre le débit imposé par le poussoir ($Q_{vérin}$) et le débit éjecté hors du récipient contenant le produit liquide à injecter ($Q_{injection}$) se crée pendant cette phase transitoire. Cet écart de débit est alors considéré comme une fuite fictive ($Q_{fuite}$).

On a alors la relation suivante :

$$Q_{injection}(t) = Q_{vérin}(t) - Q_{fuite}(t)$$

*où*

$$Q_{vérin}(t) = \frac{\omega(t)}{60} \times z \times S_{vérin} = \lambda \times \omega(t)$$

Avec :

- $Q_{injection}$ : le débit de produit liquide sortant réellement du récipient contenant le produit liquide à injecter,
- $Q_{vérin}$ : le débit imposé par le poussoir,
- $Q_{fuite}$ : le débit de fuite fictif,
- $\lambda$ : le rapport de conversion en mL par tour de moteur,
- $\omega(t)$ : la vitesse du moteur en tr/min,
- $S_{vérin}$ : la section du poussoir en mm$^2$,
- $z$ : le rapport de transmission en mm/tr.

Dès que la pression atteint un régime établi, c'est à dire que le débit imposé par le poussoir est égal au débit en sortie de seringue, alors $Q_{fuite} = 0$ et $Q_{injection}(t) = Q_{vérin}(t)$.

La loi de déformation précédemment citée permet de calculer le débit de fuite fictif en fonction de la pression dans la seringue. Il est ainsi possible, connaissant le débit imposé par le poussoir, d'estimer le débit d'injection en sortie du récipient contenant le produit liquide à injecter.

La déformation étant fonction de la pression, une caractérisation permet d'établir cette loi de déformation en fonction de la pression.

On va maintenant décrire deux exemples de méthodes permettant de déterminer la loi de déformation.

### 1.1.4.3. Détermination de la Loi de déformation

C'est grâce à la connaissance de la loi de déformation du dispositif d'injection (compliance) qu'il est possible de calculer le volume perdu en déformation (appelé volume de fuite $V_{fuite}$) pour procéder à l'estimation du débit d'injection.

Les deux méthodes suivantes peuvent être utilisées pour sa détermination.

Méthode empirique : Elle consiste à faire monter progressivement la pression dans un contenant fermé, rempli si nécessaire d'un liquide incompressible, et de relever la pression correspondant au volume de déformation obtenu. On peut alors procéder à une approximation de la courbe résultante pour obtenir une fonction qui sera connue par le dispositif d'injection.

Méthode théorique : Pour les contenants à géométrie simple il est possible d'obtenir une loi théorique. Cependant, l'avantage de la méthode empirique est la prise en compte des phénomènes complexes à modéliser par des équations mathématiques tels que la présence des jeux mécaniques à basse pression et autres non linéarités.

*1.2. Injecteur à poche*

Un inconvénient de l'utilisation d'un dispositif d'injection de type pousse-seringue tel que décrit précédemment est qu'il est nécessaire de calculer la loi de déformation pour les différents types de seringues utilisables par l'appareil. En effet, dans le cas d'un dispositif d'injection de type pousse-seringue, la loi de déformation prend en compte non seulement la déformation du dispositif d'injection, mais également la déformation de la seringue elle-même. Or cette déformation de la seringue varie d'une seringue à l'autre, notamment en fonction de ses dimensions, du matériau la constituant, etc.

L'utilisation d'un dispositif de type injecteur à poche permet de pallier cet inconvénient. En effet, dans le cas d'un injecteur de type injecteur à poche, le contenant (à savoir la poche) ne se déforme pas sous l'effet de la pression : seul le dispositif d'injection lui-même se déforme en fonction de la pression. La loi de déformation ne prend donc en compte que la déformation du dispositif d'injection.

Il est donc nécessaire de déterminer une seule loi de déformation dans le cas d'un injecteur de type injecteur à poche, et cette loi est valable quelles que soient les caractéristiques (dimensions, matériau, etc.) de la poche utilisée.

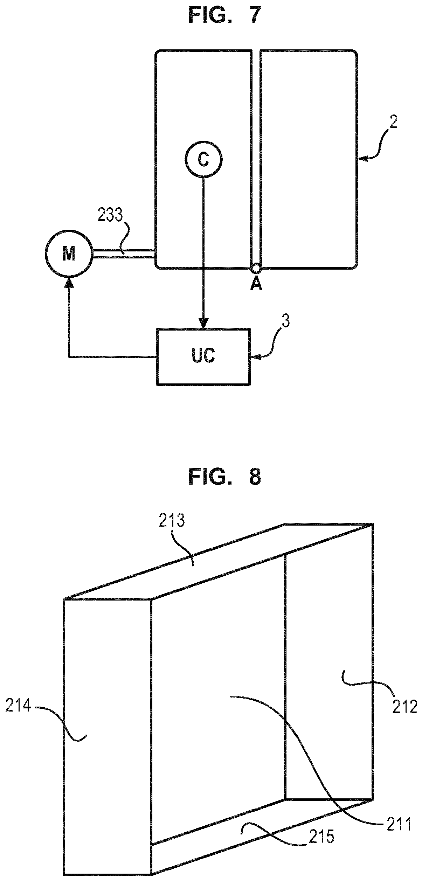

En référence à la figure 7, on a illustré un exemple de dispositif d'injection de type injecteur à poche. Le dispositif comprend une unité de mise en pression 2, une unité de contrôle 3.

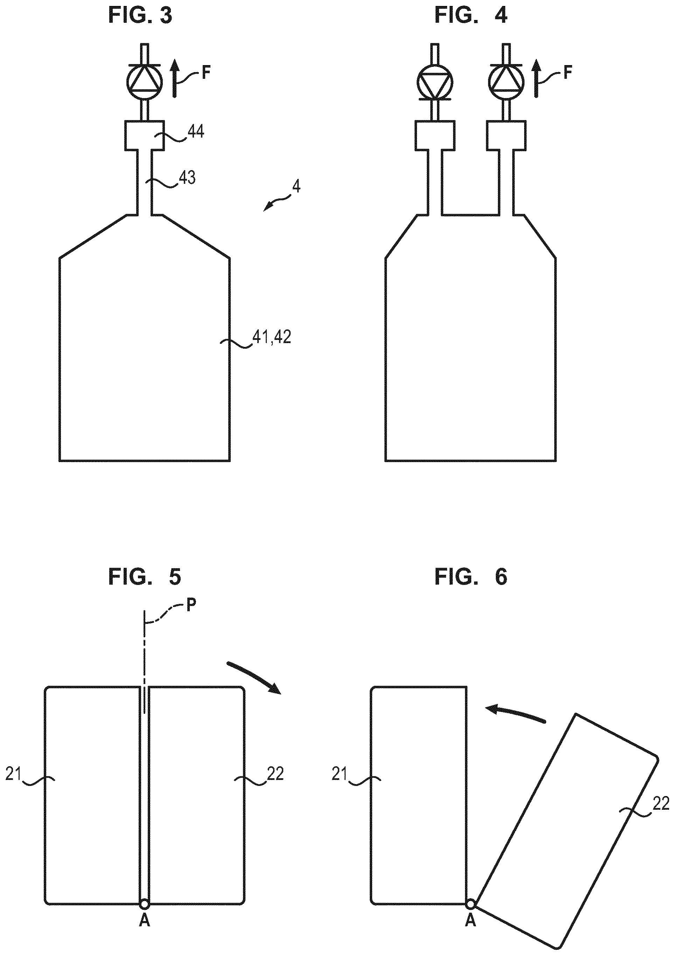

Le dispositif d'injection permet l'injection d'un produit liquide contenu dans une poche 4 (cf. figures 3 et 4).

*1.2.1. Poche*

En référence aux figures 3 et 4, la poche médicale 4 comporte deux feuilles superposées 41, 42 de longueur et de largeur appropriées ainsi qu'un (ou plusieurs) membre(s) d'accès 43.

Les feuilles 41, 42 sont faites de plusieurs couches de films minces stratifiées en matériaux flexibles ou souples, éventuellement transparent ou translucide tels que les matériaux polymères comprenant le polyéthylène, le polypropylène, et des matériaux de préférence thermoplastiques.

Les feuilles superposées 41, 42 sont de préférence soudées plates entre elles afin de former une poche 4 dont le volume est nul avant qu'elle ne soit remplie du produit liquide à injecter. Les feuilles superposées 41, 42 sont scellées à leurs périphéries latérales pour former une poche 4 d'aspect général extérieur rectangulaire. Quand la poche médicale 4 est remplie ou partiellement remplie, elle présente la forme d'un coussin.

Un membre d'accès 43 est prévu au niveau de la partie inférieure de la poche 4. Le membre d'accès 43 est scellé entre les feuilles superposées 41, 42. Ce membre d'accès 43 est un tube et peut comprendre à son extrémité distale un raccord de branchement 44 pour le couplage de la poche à une tubulure reliée au patient.

Un autre membre d'accès 43 peut être prévu sur la poche. Dans ce cas :

- le premier membre d'accès - dit membre d'accès amont - est destiné à être connecté à une source contenant le produit liquide à injecter au patient pour permettre le remplissage de la poche,
- le deuxième membre d'accès - dit membre d'accès aval - est destiné à être connecté à une tubulure reliée au patient (par l'intermédiaire de plusieurs éléments tel qu'une canalisation et un cathéter ou une aiguille hypodermique/intraveineuse) pour l'injection au patient du produit liquide.

Avantageusement, une valve anti-retour à seuil peut être positionnée entre le membre d'accès aval et la tubulure reliée au patient. La valve anti-retour à seuil est adaptée pour autoriser le passage du liquide d'amont en aval comme représenté par la flèche "F" lorsqu'une pression déterminée du fluide en écoulement est atteinte, tandis qu'elle condamne le passage du liquide médical en sens inverse, à savoir, d'aval en amont, c'est-à-dire dans le sens contraire de celui illustré par la flèche "F".

Une autre valve anti-retour peut être positionnée entre le membre d'accès amont et la source pour autoriser le passage de produit liquide uniquement de la source vers la poche.

Lorsque la poche comprend un unique membre d'accès 43, alors le membre d'accès joue les deux rôles cités précédemment, à savoir le remplissage et l'injection.

*1.2.2. Unité de mise en pression*

*1.2.2.1. Demi-coques*

L'unité de mise en pression 2 comprend une enceinte rigide composée de deux demi-coques 21, 22 articulées autour d'un axe de rotation A-A' de sorte à permettre le déplacement relatif des demi-coques l'une par rapport à l'autre. Ces deux demi-coques 21, 22 sont aptes à être déplacées relativement l'une par rapport à l'autre entre :

- une position ouverte (figure 6) pour la mise en place de la poche et
- une position fermée (figure 5) pour l'injection du produit liquide contenu dans la poche.

De préférence l'une des demi-coques 21 est fixe et l'autre 22 est mobile en rotation selon l'axe A-A'.

Avantageusement, l'axe de rotation A-A' peut être décalé par rapport à un plan P de fermeture passant par les zones de contact entre les deux demi-coques 21, 22 lorsque celles-ci sont dans la position fermée. Par exemple, l'axe de rotation A-A' peut être positionné sous la demi-coque fixe 21. Ceci permet d'obtenir une ouverture automatique de la demi-coque mobile 22 par gravité afin de limiter le nombre de manipulations nécessaires par l'utilisateur.

De préférence, les demi-coques 21, 22 ne sont pas motorisées afin d'éviter les risques de pincement de l'utilisateur. Des ressorts (non représentés) peuvent être prévus entre les deux demi-coques pour assister l'utilisateur dans la fermeture de la porte mobile par compensation du poids de la demi-coque mobile.

Chaque demi-coque comporte une cavité de sorte à former un berceau.

Dans le mode de réalisation illustré à la figure 8, chaque demi-coque comporte 21, 22 une paroi de fond 211, une paroi de face et quatre parois latérales 212 à 215 s'étendant à la périphérie de la paroi de fond 211, perpendiculairement à celle-ci. La paroi de face est destinée à venir en contact avec une poche. La paroi de face peut présenter une forme concave de sorte à définir la cavité formant le berceau. En variante, l'une ou chaque demi-coque peut être monobloc et comporter une unique paroi concave définissant la cavité formant le berceau.

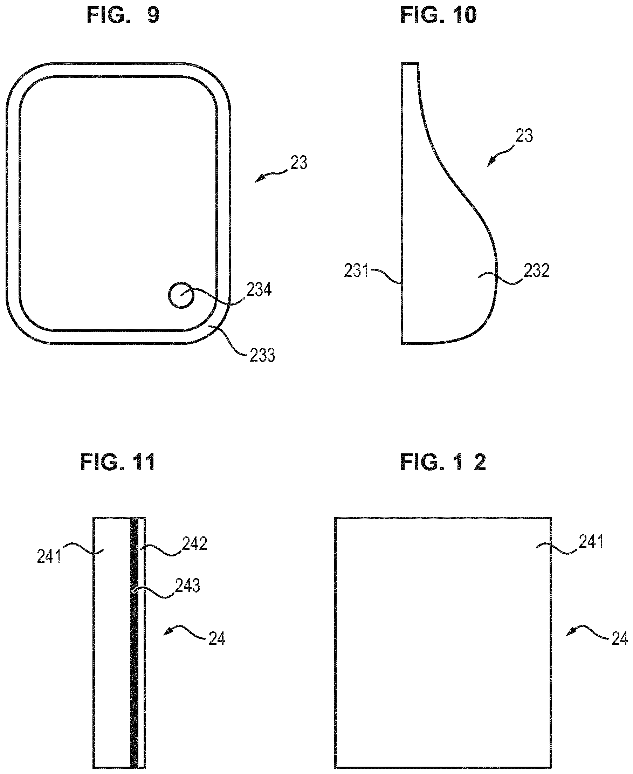

La cavité de l'une 21 des demi-coques - par exemple la demi-coque fixe - est destinée à recevoir une vessie 23 illustrée aux figures 9 et 10. La vessie 23 est composée d'au moins deux membranes 231, 232 soudées à leur périphérie. Ces membranes 231, 232 soudées forment un espace destiné à recevoir un fluide moteur provoquant une variation du volume de la vessie 23 afin d'induire une déformation de celle-ci. L'alimentation de la vessie 23 en fluide moteur est réalisée grâce à un vérin hydraulique M connecté à la vessie 23 par l'intermédiaire de flexibles d'alimentation hydraulique 233.

La cavité de l'autre 22 des demi-coques - par exemple la demi-coque mobile en rotation - est destinée à recevoir un coussin amortisseur déformable à volume constant 24. Ce coussin 24 est dit « coussin passif » en ce qu'il n'est pas alimenté en fluide moteur. Sa déformation est liée aux forces appliquées sur celui-ci. Le fait que la demi-coque mobile 22 soit agencée pour recevoir un coussin passif 24 permet d'éviter la présence d'une alimentation hydraulique (pour le passage du fluide moteur) sur la demi-coque mobile. Ceci permet de diminuer l'encombrement du dispositif d'injection puisqu'il n'est pas nécessaire de prévoir un dégagement pour que des flexibles d'alimentation hydrauliques 233 puissent se déplacer avec la demi-coque mobile 22. Ceci permet également de faciliter la manipulation du dispositif d'injection puisqu'il n'est plus nécessaire pour l'utilisateur d'assister le déplacement des flexibles d'alimentation hydraulique.

Les demi-coques 21, 22 peuvent être réalisées, par exemple, en aluminium ou en matériau composite avec de la fibre de verre ou de la fibre de carbone.

Avantageusement, les demi-coques 21, 22 peuvent s'ouvrir dans deux positions :

- une première position de préparation où les deux demi-coques forment un angle compris entre 10° et 45° l'une par rapport à l'autre ; cette première position d'ouverture permet l'insertion d'une poche dans le dispositif d'injection,
- une deuxième position de maintenance où les deux demi-coques forment un angle compris entre 85 et 95°l'une par rapport à l'autre, et de préférence égal à 90° ; cette deuxième position d'ouverture permet le nettoyage du dispositif d'injection.

*1.2.2.2. Vessie*

L'une des membranes 232 de la vessie 23 - dite « membrane de fond » - est destinée à venir en regard de la paroi de fond 211 de la demi-coque.

De préférence, la forme de la membrane de fond 232 est complémentaire de la forme de la paroi de fond 211 de la demi-coque. Par exemple, dans un mode de réalisation la membrane de fond 232 et la paroi de fond 211 sont en forme de goutte (cf. figure 10). Ceci permet de limiter la quantité de fluide moteur à introduire dans (respectivement extraire de) la vessie 23 pour augmenter (respectivement diminuer) son volume. On limite ainsi l'encombrement du dispositif d'injection, et on améliore la réactivité du dispositif à vitesse de remplissage donnée.

L'autre membrane 231 - dite membrane frontale - est destinée à venir en regard de la poche 4.

La rigidité de la membrane de fond 232 peut être prévue supérieure à la rigidité de la membrane frontale 231. Par exemple :

- la membrane frontale 231 peut être souple et présenter une dureté shore de l'ordre de 30 à 40 shore,
- la membrane de fond 232 peut être semi-rigide et présenter une dureté shore de l'ordre de 80 shore.

Le fait que la membrane de fond 232 présente une rigidité supérieure à la membrane frontale 231 permet :

- d'une part d'obtenir un bon placage de la membrane de fond 232 contre la paroi de fond 211 de la demi-coque même à faible pression,
- d'autre part de garantir que lors de l'introduction de fluide moteur dans la vessie 23, ce soit la paroi frontale 231 de la vessie 23 qui se déforme.

La vessie 23 comprend également une ouverture 234 - par exemple dans la membrane de fond 232 - pour le passage du fluide moteur. L'introduction (respectivement le retrait) de fluide moteur dans (respectivement de) la vessie 23 induit une variation (augmentation ou diminution) de son volume qui provoque une déformation de celle-ci.

Enfin, la vessie 23 comprend un corps annulaire et rigide de renfort 233. Ce corps de renfort 233 s'étend à la périphérie des membranes de la vessie 23. Ce corps de renfort 233 est par exemple en métal ou en dacron. La présence d'un corps de renfort 233 à la périphérie des membranes de la vessie 23 permet d'éviter la formation d'une hernie (i.e. bourrelet) entre les deux demi-coques 21, 22 durant l'introduction de fluide moteur dans la vessie lorsque les demi-coques sont dans la position fermée.

### 1.2.2.3. *Coussin*

En référence aux figures 11 et 12, le coussin passif 24 comprend une couche épaisse souple 241. Cette couche épaisse 241 est de préférence constituée dans un matériau de dureté shore nulle, et éventuellement de forte conductivité thermique.

Le matériau constituant la couche épaisse 241 est, par exemple, du silicone ou du polyuréthane.

Lorsque la couche épaisse 241 est en silicone, celle-ci peut être recouverte d'une couche mince anti-adhérente pour limiter les frottements entre la poche et le coussin passif 24. Cette couche mince est par exemple une couche de peinture polyuréthane ou une enveloppe de coton ou de Lycra®.

Le coussin passif 24 peut comprendre une face arrière rigide 242 destinée à venir en regard de la paroi de fond 211 de la demi-coque. Dans ce cas, la face arrière rigide 242 présente une forme conjuguée de la forme de la paroi de fond 211 de la demi-coque. La présence d'une face arrière rigide 242 sur le coussin 24 permet de faciliter sa manipulation.

Le coussin passif 24 peut également comprendre un (ou plusieurs) élément(s) chauffant 243 composé(s) par exemple d'une couche isolante et d'une couche résistive, ou tout autre type d'élément chauffant connu de l'homme du métier. La présence d'un élément chauffant 243 permet de maintenir le produit liquide contenu dans la poche à une température désirée préalablement à son injection dans le patient.

De préférence, l'élément chauffant 243 est positionné entre la face arrière rigide 242 et la couche épaisse souple 241. En effet :

- l'élément chauffant étant non extensible, et
- la couche épaisse étant destinée à être déformée,

il est préférable de positionner l'élément chauffant entre la face arrière et la couche épaisse pour limiter les risques de détérioration de celui-ci.

### 1.2.2.4. *Logement d'embout de poche*

Le dispositif d'injection peut également comprendre un logement d'embout de poche sur l'une des demi-coques 21, 22. Ce logement est destiné à recevoir le raccord de branchement du membre d'accès 43 de la poche 4, ou tout type d'élément de couplage positionné à l'extrémité distale du membre d'accès 43.

La forme de ce logement présente de préférence une symétrie de révolution. Ceci permet d'assurer un bon positionnement de la poche dans l'unité de mise en pression par gravité sans requérir une attention particulière de la part de l'utilisateur. Le logement présente par exemple la forme d'un tronc de cône (tel un entonnoir) ou encore d'un cylindre.

Le logement présente en outre une fente longitudinale sur sa face interne. Cette fente est destinée à recevoir l'extrémité d'une tubulure couplée au membre d'accès. Ceci permet d'assurer un bon positionnement de la tubulure le long du dispositif d'injection.

Un capteur de bulle peut être prévu au niveau de cette fente longitudinale pour permettre la détection de bulles dans la tubulure reliée au patient.

### 1.2.3. *Unité de contrôle*

L'unité de contrôle comprend les mêmes éléments que dans la variante de réalisation pousse-seringue, à savoir notamment un calculateur. Ces éléments ne seront pas décrits à nouveau ici.

### 1.2.3.1. *Estimation du débit de sortie de l'injecteur*

La technologie de compression hydraulique permet une injection continue et sans pulsation pour l'ensemble du produit liquide injectable contenu dans la poche. Lorsque la poche de produit liquide injectable est bien plaquée contre la membrane frontale de la vessie et la couche épaisse du coussin, tout déplacement relatif de fluide moteur par le vérin hydraulique vers l'enceinte rigide induit le même déplacement de produit liquide injectable. C'est le principe de la conservation du débit. Ainsi, une injection de 80 mL de produit liquide injectable à 3mL/s sera obtenue par une introduction de 80mL de fluide incompressible moteur à un débit de 3mL/s.

Cependant, il n'existe pas de fluide parfaitement incompressible, ni de matériau indéformable. Une compensation est donc nécessaire pour accélérer la mise en place de ces phénomènes et arriver rapidement au régime établi.

De plus, dans le cas réel, les écoulements ne sont pas parfaits : de nombreux frottements opèrent dans les tubulures. Ces frottements sont fonction de la viscosité des produits liquides injectables, du débit d'injection, des caractéristiques des tubulures (rugosité, diamètre intérieur,...) et des cathéters. Ces frottements, appelés « pertes de charge », se traduisent par une montée en pression dans l'enceinte rigide. L'enceinte est susceptible de se déformer sous l'effet de la pression, créant ainsi, pendant toute la durée de la montée en pression (régime transitoire), un débit de fuite fictif entre le débit imposé par le vérin hydraulique et le débit de produit liquide réellement éjecté hors de la poche.

On a alors la relation suivante :

$$Q_{injection}(t) = Q_{vérin}(t) - Q_{fuite}(t)$$

*où*

$$Q_{vérin}(t) = \frac{\omega(t)}{60} \times z \times S_{vérin} = \lambda \times \omega(t)$$

Avec :

- $Q_{injection}$ : le débit de produit liquide sortant réellement du dispositif,
- $Q_{vérin}$ : le débit imposé par le vérin hydraulique,
- $Q_{fuite}$ : le débit de fuite fictif,
- $\lambda$ : le rapport de conversion en mL/tr,
- $\omega(t)$ : la vitesse du moteur entraînant le vérin hydraulique en tr/min,
- $S_{vérin}$ : la section du vérin hydraulique en mm$^2$,
- $z$ : le rapport de transmission en mm/tr.

Dès que la pression atteint un régime établi, c'est à dire que le débit en sortie du vérin hydraulique est égal au débit en sortie de la poche, alors $Q_{fuite} = 0$ et $Q_{injection}(t) = Q_{vérin}(t)$.

$Q_{fuite}$ est la dérivée par rapport au temps du volume de déformation de l'enceinte noté $V_{fuite}$.

Cette loi de déformation est elle-même fonction de la pression. Comme décrit précédemment, une caractérisation permet d'établir cette loi de déformation en fonction de la pression. On obtient alors :

$$V_{fuite} = f(p)$$

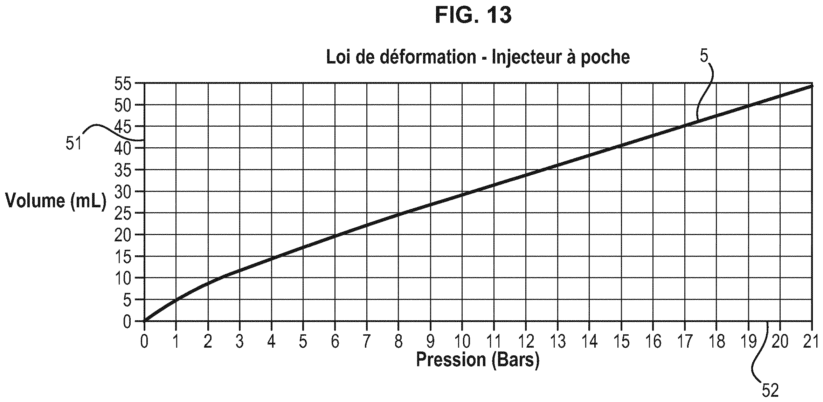

En référence à la figure 13, on a illustré un exemple de loi de déformation 5 permettant de déterminer un volume de fuite 51 en fonction de la pression dans l'enceinte 52. La pression dans l'enceinte peut être mesurée par exemple en disposant un capteur de pression C dans la vessie 23.

On peut donc calculer en temps réel $Q_{injection}(t)$ et réaliser un asservissement de l'avancée du vérin hydraulique.

### 2. *Asservissement de l'unité de mise en pression en fonction du débit de sortie*

L'asservissement de l'unité de mise en pression peut être réalisé de manière analogue pour les deux variantes de réalisation (i.e. pousse-seringue et injecteur à poche) décrites ci-dessus.

Dans la suite, on présentera le principe d'asservissement de l'unité de mise en pression en référence au mode de réalisation de type injecteur à poche, étant entendu que ce principe peut être transposé au dispositif d'injection de type pousse-seringue.

Lorsque l'enceinte est mise sous pression, elle tend à se déformer. Cette déformation n'est pas instantanée et dure jusqu'à ce que la pression d'injection ait atteint sa valeur définitive. On peut alors parler du régime établi.

Lorsque le dispositif d'injection n'est pas asservi, le temps de stabilisation est bien trop long pour pouvoir garantir que le volume injecté et la durée de l'injection restent compris dans des tolérances acceptables par l'utilisateur.

Il est donc possible de réguler le débit de sortie du dispositif d'injection en agissant sur la commande appliquée au vérin.

Cette régulation ou asservissement peut utiliser un régulateur de type PI (Proportionnel Intégral) - ci-après dénommé « régulateur PI » - pour accélérer la montée en pression tout en respectant un dépassement minimal du débit de consigne saisi par l'utilisateur.

Les paramètres du régulateur s'adaptant à une seule configuration du dispositif d'injection, un jeu de paramètres ne suffit pas pour pouvoir couvrir toutes les gammes de débit et de configurations du dispositif d'injection (i.e. type de produit liquide injectable, tubulures du raccordement au patient, cathéters, etc.).

Une méthode qui permet d'avoir un régulateur PI auto-ajustable en fonction du contexte de l'injection a été mis au point sur la base des fonctions qui décrivent les paramètres à utiliser en fonction :

- de la pression instantanée dans le récipient contenant le produit liquide injectable, et
- du débit de consigne saisi par l'utilisateur sur des moyens de saisie (tel qu'un clavier, un écran tactile, etc.) du dispositif d'injection.

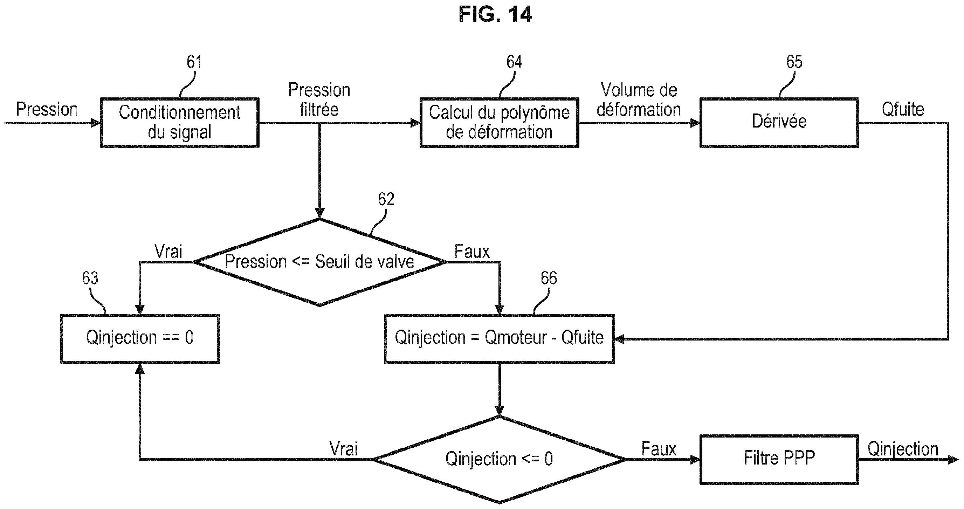

Ces fonctions sont illustrées à la figure 14. En fonction d'un débit de consigne 53 et d'une pression dans le récipient 54, il est possible de déterminer :

- un terme proportionnel « Kp » à partir de la fonction proportionnelle 55,
- un terme intégral « Ki » à partir de la fonction intégrale 56.

Comme on peut le constater sur les fonctions proportionnelle et intégrale 55, 56, plus la pression dans l'enceinte augmente, plus le terme proportionnel Kp doit être important pour compenser la déformation rapide de l'enceinte. En revanche, ce terme se voit décrémenté pour les injections à haut débit. Pour le terme intégral Ki, on remarque la présence d'un plafond à haute pression et haut débit, ce qui permet de ne pas cumuler une erreur trop importante lors d'une montée très lente en pression de l'enceinte. Le terme intégral Ki décroît exponentiellement en fonction de la pression et garde une valeur réduite à haut débit, même si la pression est importante.

Ces courbes Ki et Kp peuvent être obtenues par calibration manuelle du PI sur le système physique avec des méthodes standard de réglage (connus sous le nom anglo-saxon de « tuning »),

Le tableau ci-dessous donne des exemples de valeurs pour le terme intégral Ki et le terme proportionnel Kp pour différentes valeurs de débit de consigne et pression mesurée à l'intérieur du récipient.

| Ki | | Pression (bar) | | | | | | | | | | |
|---|---|---|---|---|---|---|---|---|---|---|---|---|
| | | **0** | **2** | **4** | **6** | **8** | **10** | **12** | **14** | **16** | **18** | **20** |
| Débit (mL/s) | **0** | 2.53 | 0.994 | 0.729 | 0.698 | 0.692 | 0.691 | 0.691 | 0.692 | 0.693 | 0.693 | 0.694 |
| | **2** | 2.53 | 1.465 | 0.798 | 0.72 | 0.7 | 0.694 | 0.691 | 0.691 | 0.691 | 0.691 | 0.692 |
| | **4** | 2.53 | 2.53 | 0.968 | 0.777 | 0.725 | 0.706 | 0.697 | 0.693 | 0.692 | 0.691 | 0.691 |
| | **6** | 2.53 | 2.53 | 1.399 | 0.916 | 0.789 | 0.74 | 0.717 | 0.705 | 0.699 | 0.695 | 0.693 |
| | **8** | 2.53 | 2.53 | 2.53 | 1.263 | 0.945 | 0.825 | 0.769 | 0.739 | 0.721 | 0.711 | 0.704 |
| | **10** | 2.53 | 2.53 | 2.53 | 2.208 | 1.339 | 1.035 | 0.897 | 0.823 | 0.78 | 0.753 | 0.735 |

| Kp | Pression (bar) | | | | | | | | | | |
|---|---|---|---|---|---|---|---|---|---|---|---|
| | | **0** | **2** | **4** | **6** | **8** | **10** | **12** | **14** | **16** | **18** | **20** |
| Débit (mL/s) | **0** | 1.202 | 1.722 | 4.252 | 6.752 | 8.893 | 10.911 | 12.947 | 15.035 | 17.158 | 19.284 | 21.384 |
| | **2** | 1.202 | 1.332 | 2.725 | 4.667 | 6.458 | 8.045 | 9.521 | 10.966 | 12.424 | 13.909 | 15.42 |
| | **4** | 1.202 | 1.221 | 1.781 | 3.019 | 4.423 | 5.754 | 6.967 | 8.087 | 9.151 | 10.191 | 11.229 |
| | **6** | 1.202 | 1.203 | 1.353 | 1.943 | 2.844 | 3.85 | 4.846 | 5.788 | 6.668 | 7.495 | 8.283 |
| | **8** | 1.202 | 1.202 | 1.225 | 1.414 | 1.845 | 2.453 | 3.151 | 3.878 | 4.597 | 5.291 | 5.953 |
| | **10** | 1.202 | 1.202 | 1.204 | 1.239 | 1.376 | 1.642 | 2.019 | 2.471 | 2.969 | 3.487 | 4.01 |

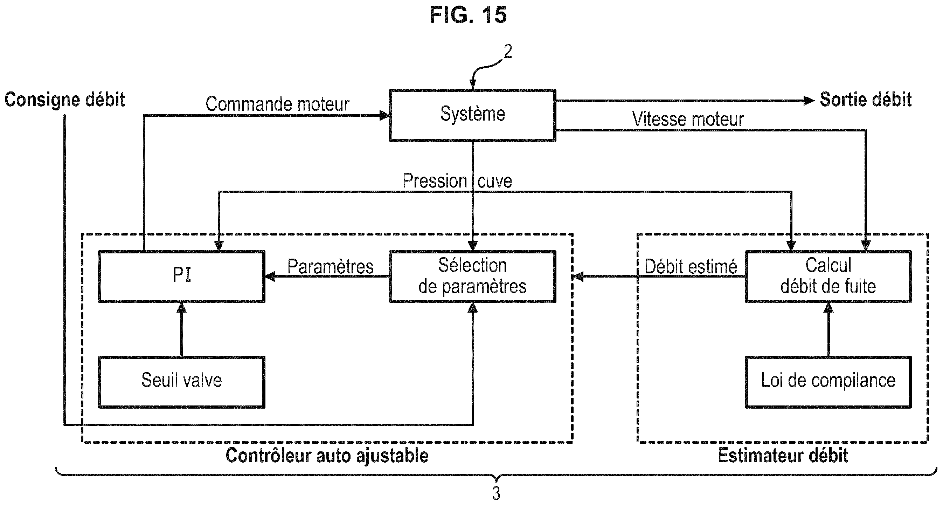

On va maintenant décrire le principe de fonctionnement du dispositif d'injection selon l'invention en référence aux figures 14 et 15.

L'utilisateur déverrouille les moyens de pompage du dispositif. La demi-coque mobile se déplace en rotation autour de l'axe de rotation A-A' jusqu'à la position ouverte (figure 6) de mise en place de la poche 4. L'utilisateur insère le membre d'accès 43 dans la fente longitudinale du logement d'embout, le raccord de branchement étant positionné vers le haut et la poche vers le bas. Du fait de la symétrie de révolution du logement d'embout, la poche se positionne correctement entre les deux demi-coques sous l'effet de la gravité.

L'utilisateur referme ensuite l'enceinte en ramenant la demi-coque mobile dans une position verticale. Lorsque les deux demi-coques sont en contact, des moyens de verrouillage s'actionnent soit automatiquement, soit manuellement.

L'unité de contrôle commande le déplacement du vérin hydraulique pour gonfler la vessie de la demi-coque fixe afin d'assurer un bon placage de la vessie et du coussin passif contre les faces de la poche 4. Du fait de la présence de la valve à seuil en sortie du dispositif d'injection, aucun produit liquide injectable n'est expulsé de la poche avant que les faces de la poche ne soient plaquées contre la vessie d'une part et le coussin d'autre part.

L'utilisateur saisit ensuite sur un écran tactile du dispositif d'injection des paramètres d'injection, et notamment un débit de consigne. Ce débit de consigne est reçu par l'unité de contrôle qui déclenche le protocole d'injection.

Le calculateur reçoit en temps réel la pression mesurée par le capteur de pression positionné dans la vessie, comme illustré à la figure 15 (étape 61). Le calculateur reçoit également un débit moteur correspondant au débit de fluide moteur déplacé par le vérin hydraulique et mesuré par toute technique connue de l'homme du métier.

Le calculateur compare la pression mesurée à la pression d'ouverture de la valve à seuil (étape 62). Si cette pression mesurée est inférieure à la pression d'ouverture, alors le débit de sortie est nul (étape 63).

Sinon, le calculateur estime le volume de fuite (ou volume de déformation de l'enceinte) à partir de la pression mesurée et de la fonction de déformation (étape 64). Le calculateur dérive ensuite par rapport au temps ce volume de fuite calculé pour obtenir le débit de fuite fictif du dispositif (étape 65).

Ce débit de fuite fictif est ensuite soustrait au débit moteur mesuré pour obtenir le débit estimé d'injection en sortie de l'enceinte (étape 66).

Ce débit d'injection estimé sera utilisé par le calculateur pour le comparer au débit de consigne. L'écart entre ces deux débits constitue l'erreur à corriger par le calculateur au moyen du régulateur PI.

A partir du débit de consigne, de la pression mesurée dans l'enceinte et des courbes proportionnelle et intégrale, le calculateur détermine les paramètres de régulation Ki et Kp.

Au moyen du régulateur PI, le calculateur convertit les paramètres Ki et Kp et l'erreur à corriger en une commande moteur.

### 3. *Autres fonctions de l'unité de contrôle*

Outre l'estimation du débit de sortie et l'asservissement du dispositif d'injection, l'unité de contrôle permet une vérification de la cohérence entre la nature du produit liquide contenu dans l'unité de mise en pression et une information saisie par l'utilisateur.

En effet, la nature du produit liquide présent dans la poche peut être déterminée grâce à une analyse de son comportement dans l'enceinte. En effet, la montée en pression de l'enceinte est différente selon la viscosité du liquide entraîné dans les tubulures.

Par exemple, un produit liquide de type produit de contraste aura une montée en pression plus progressive qu'un produit liquide de type sérum physiologique. S'il y a une différence de comportement entre le comportement attendu du produit liquide dont la nature a été saisie par l'utilisateur et le comportement du produit liquide réellement présent dans l'enceinte, alors l'erreur de manipulation peut être détectée. L'unité de contrôle peut donc être programmée pour :

- recevoir une information sur la nature du produit liquide contenu dans la poche, cette information étant saisie par l'utilisateur sur les moyens de saisie du dispositif d'injection,
- estimer le débit de sortie du dispositif d'injection,
- comparer le débit de sortie attendu au débit de sortie estimé,
- émettre une alarme si la différence entre le débit de sortie estimé et le débit de sortie de référence est supérieure à un seuil.

Le lecteur aura compris que de nombreuses modifications peuvent être apportées sans sortir matériellement des nouveaux enseignements et des avantages décrits ici. Par exemple, dans le cas d'une unité de mise en pression de type injecteur à poche, le dispositif d'injection peut comprendre deux paires de demi-coquille permettant l'injection successive ou simultanée de différents produits liquides injectables.

**Revendications**

1. Système d'injection à un patient d'un produit liquide contenu dans un récipient (4), le système comprenant une unité de mise en pression (2) pour l'entrainement du produit liquide contenu dans le récipient, et un calculateur programmé pour mettre en oeuvre les étapes suivantes :

   - Recevoir une information représentative d'un débit réel d'avancement de l'unité de mise en pression, ledit débit réel d'avancement étant le débit généré par l'unité de mise en pression (2) en fonction d'un débit de consigne saisi par l'utilisateur,
   - Recevoir une information représentative d'une pression à l'intérieur du récipient,

   le système étant **caractérisé en ce que** ledit calculateur est programmé en outre pour mettre en oeuvre les étapes suivantes :

   - Estimer un débit de fuite fictif en fonction d'une loi de déformation du système d'injection sous l'effet de la pression à l'intérieur du récipient,
   - Estimer en continu un débit de sortie du récipient correspondant à la différence entre le débit réel d'avancement de l'unité de mise en pression et le débit de fuite fictif.

2. Système d'injection selon la revendication 1, dans lequel le calculateur estime le débit de fuite fictif en fonction de la pression à l'intérieur du récipient.

3. Système d'injection selon l'une quelconque des revendications 1 ou 2, dans lequel l'unité de mise en pression (2) est constituée d'au moins une enceinte destinée à recevoir une poche contenant le produit liquide à injecter et comprenant au moins un orifice d'écoulement destiné à être connecté à une tubulure reliée au patient par l'intermédiaire d'un conduit d'injection, l'enceinte comportant au moins une membrane déformable à volume variable sous l'action d'un fluide moteur injecté dans ladite enceinte de sorte à comprimer la poche afin de forcer le produit liquide à injecter à s'écouler dans la tubulure.

4. Système selon l'une quelconque des revendications précédentes, dans lequel le calculateur inclut un régulateur auto-ajustable - tel qu'un régulateur de type proportionnel intégral - le calculateur ajustant automatiquement des paramètres du régulateur - tels que des coefficients Ki, Kp - en fonction de la pression à l'intérieur du récipient et du débit de consigne saisi par l'utilisateur.

5. Système selon l'une quelconque des revendications précédentes, dans lequel le calculateur est programmé pour déterminer un débit de sortie de référence en fonction d'une information relative à la nature du produit liquide contenu dans le récipient.

6. Système selon la revendication précédente, dans lequel le calculateur est programmé pour :

   - comparer le débit de sortie de référence au débit de sortie estimé et
   - émettre une alarme si la différence entre le débit de sortie de référence et le débit de sortie estimé est supérieure à une valeur seuil.

7. Système selon l'une quelconque des revendications précédentes, lequel comprend en outre un afficheur pour l'affichage d'un graphique représentant un débit de sortie du récipient en fonction du temps, le débit de sortie étant égal à la différence entre le débit de consigne et le débit de fuite fictif estimé.

8. Système selon l'une quelconque des revendications précédentes, dans lequel le produit liquide est un produit de contraste.

9. Procédé de contrôle d'un système d'injection à un patient d'un produit liquide contenu dans un récipient, le procédé comprenant les étapes suivantes :

- Commander l'entrainement du produit liquide contenu dans le récipient à l'aide d'une unité de mise en pression (2) du système d'injection,
- Recevoir une information représentative d'un débit réel d'avancement de l'unité de mise en pression, ledit débit réel d'avancement étant le débit généré par l'unité de mise en pression (2) en fonction d'un débit de consigne saisi par l'utilisateur,
- Recevoir une information représentative d'une pression à l'intérieur du récipient,

**caractérisé en ce que** le procédé comprend en outre les étapes suivantes :

- Estimer un débit de fuite fictif en fonction d'une loi de déformation du système d'injection sous l'effet de la pression à l'intérieur du récipient,
- Estimer en continu d'un débit de sortie du récipient correspondant à la différence entre le débit réel d'avancement de l'unité de mise en pression et le débit de fuite fictif.

## Patentansprüche

1. System zur Injektion eines in einem Behälter (4) enthaltenen flüssigen Produkts in einen Patienten, umfassend eine Druckeinheit (2) für den Vorschub des in dem Behälter enthaltenen flüssigen Produkts und einen Rechner, der programmiert ist, die folgenden Schritte einzusetzen:

- Empfangen einer Information, die für eine tatsächliche Vorschubmenge der Druckeinheit repräsentativ ist, wobei die tatsächliche Vorschubmenge die von der Druckeinheit (2) in Abhängigkeit von einer vom Benutzer erfassten Sollmenge erzeugte Menge ist,
- Empfangen einer Information, die für einen Druck im Inneren des Behälters repräsentativ ist,

das System **dadurch gekennzeichnet ist, dass** der Rechner ferner programmiert ist, um die folgenden Schritte einzusetzen:

- Schätzen einer fiktiven Leckagemenge in Abhängigkeit von einem Verformungsgesetz des Injektionssystems unter der Wirkung des Drucks im Inneren des Behälters,
- kontinuierliches Schätzen einer Austrittsmenge aus dem Behälter entsprechend der Differenz zwischen der tatsächlichen Vorschubmenge der Druckeinheit und der fiktiven Leckagemenge.

2. Injektionssystem nach Anspruch 1, bei dem der Rechner die fiktive Leckagemenge in Abhängigkeit vom Druck im Inneren des Behälters schätzt.

3. Injektionssystem nach einem der Ansprüche 1 oder 2, bei dem die Druckeinheit (2) aus mindestens einem Raum besteht, der dazu bestimmt ist, eine Tasche, die das zu injizierende flüssige Produkt enthält, aufzunehmen, und umfassend mindestens eine Abflussöffnung, die dazu bestimmt ist, an eine Röhre angeschlossen zu werden, die mit dem Patienten über eine Injektionsleitung verbunden ist, wobei der Raum mindestens eine verformbare Membran mit variablem Volumen unter der Wirkung eines in den Raum injizierten Vorschubfluids umfasst, um die Tasche zusammenzudrücken, um das zu injizierende flüssige Produkt dazu zu zwingen, in die Röhre abzufließen.

4. System nach einem der vorhergehenden Ansprüche, bei dem der Rechner einen selbstjustierenden Regler - wie einen Integral-Proportional-Regler - einschließt, wobei der Rechner automatisch Parameter des Reglers - wie Koeffizienten Ki, Kp - in Abhängigkeit vom Druck im Inneren des Behälters und von der vom Benutzer erfassten Sollmenge justiert.

5. System nach einem der vorhergehenden Ansprüche, bei dem der Rechner programmiert ist, um eine Referenzaustrittsmenge in Abhängigkeit von einer Information zur Natur des in dem Behälter enthaltenen flüssigen Produkts zu bestimmen.

6. System nach dem vorhergehenden Anspruch, bei dem der Rechner programmiert ist, um:

- die Referenzaustrittsmenge mit der geschätzten Austrittsmenge zu vergleichen, und
- einen Alarm zu entsenden, wenn die Differenz zwischen der Referenzaustrittsmenge und der geschätzten Austrittsmenge größer als ein Grenzwert ist.

7. System nach einem der vorhergehenden Ansprüche, das ferner eine Anzeigeeinheit für die Anzeige einer Grafik umfasst, die eine Austrittsmenge aus dem Behälter in Abhängigkeit von der Zeit darstellt, wobei die Austrittsmenge gleich der Differenz zwischen der Sollmenge und der geschätzten fiktiven Leckagemenge ist.

8. System nach einem der vorhergehenden Ansprüche, bei dem das flüssige Produkt ein Kontrastmittel ist.

9. Verfahren zur Kontrolle eines Systems zur Injektion eines in einem Behälter enthaltenen flüssigen Produkts in einen Patienten, wobei das Verfahren die folgenden Schritte umfasst:

- Steuern des Vorschubs des in dem Behälter enthaltenen flüssigen Produkts mit Hilfe einer Druckeinheit (2) des Injektionssystems,
- Empfangen einer Information, die für eine tatsächliche Vorschubmenge der Druckeinheit repräsentativ ist, wobei die tatsächliche Vorschubmenge die von der Druckeinheit (2) in Abhängigkeit von einer vom Benutzer erfassten Sollmenge erzeugte Menge ist,
- Empfangen einer Information, die für einen Druck im Inneren des Behälters repräsentativ ist,

**dadurch gekennzeichnet, dass** das Verfahren ferner die folgenden Schritte umfasst:

- Schätzen einer fiktiven Leckagemenge in Abhängigkeit von einem Verformungsgesetz des Injektionssystems unter der Wirkung des Drucks im Inneren des Behälters,
- kontinuierliches Schätzen einer Austrittsmenge aus dem Behälter entsprechend der Differenz zwischen der tatsächlichen Vorschubmenge der Druckeinheit und der fiktiven Leckagemenge.

**Claims**

1. System for injection into a patient of a liquid product contained in a container (4), the system comprising a pressurization unit (2) for driving the liquid product contained in the container, and a computer programmed to implement the following steps:

- receiving information representative of a real flow rate of advance of the pressurization unit, said real flow rate of advance being the flow rate generated by the pressurization unit (2) as a function of a set point flow rate entered by the user,
- receiving information representative of a pressure inside the container,

the system being **characterized in that** said computer is further programmed to implement the following steps:

- estimating an imaginary leakage flow rate as a function of a law of deformation of the injection system under the effect of the pressure inside the container,
- continuously estimating an outlet flow rate from the container corresponding to the difference between the real flow rate of advance of the pressurization unit and the imaginary leakage flow rate.

2. Injection system according to Claim 1, wherein the computer estimates the imaginary leakage flow rate as a function of the pressure inside the container.

3. Injection system according to either one of Claims 1 and 2, wherein the pressurization unit (2) is composed of at least one enclosure designed to receive a sachet containing the liquid product to be injected and comprising at least one flow orifice designed to be connected to a tube connected to the patient via an injection pipe, the enclosure including at least one variable volume membrane deformable as a result of the action of a driving fluid injected into said enclosure so as to compress the sachet in order to force the liquid product to be injected to flow in the tube.

4. System according to any one of the preceding claims, wherein the computer includes a self-adjusting regulator - such as a regulator of proportional-integral type - the computer automatically adjusting parameters of the regulator - such as coefficients Ki, Kp - as a function of the pressure inside the container and the set point flow rate entered by the user.

5. System according to any one of the preceding claims, wherein the computer is programmed to determine a reference outlet flow rate as a function of information relating to the nature of the liquid product contained in the container.

6. System according to the preceding claim, wherein the computer is programmed:

   - to compare the reference outlet flow rate to the estimated outlet flow rate, and
   - to emit an alarm if the difference between the reference outlet flow rate and the estimated outlet flow rate is above a threshold value.

7. System according to any one of the preceding claims, further comprising a display for displaying a graph representing a outlet flow rate from the container as a function of time, the outlet flow rate being equal to the difference between the set point flow rate and the estimated imaginary leakage flow rate.

8. System according to any one of the preceding claims, wherein the liquid product is a contrast product.

9. Method of controlling a system of injection into a patient of a liquid product contained in a container, the method comprising the following steps:

   - controlling the driving of the liquid product contained in the container using a pressurization unit (2) of the injection system,
   - receiving information representative of a real flow rate of advance of the pressurization unit, said real flow rate of advance being the flow rate generated by the pressurization unit (2) as a function of a set point flow rate entered by the user,
   - receiving information representative of a pressure inside the container,
   - **characterized in that** the method further comprises the following steps:

       - estimating an imaginary leakage flow rate as a function of a law of deformation of the injection system under the effect of the pressure inside the container,
       - continuously estimating of an outlet flow rate from the container corresponding to the difference between the real flow rate of advance of the pressurization unit and the imaginary leakage flow rate.

FIG. 1

FIG. 2

FIG. 3

44

43

4

41,42

F

FIG. 4

F

FIG. 5

P

21

22

A

FIG. 6

21

22

A

## FIG. 7

## FIG. 8

FIG. 9

FIG. 10

23

234

233

23

231

232

FIG. 11

FIG. 1 2

241

242

243

24

241

24

EP 2 861 278 B1

## FIG. 13

Loi de déformation - Injecteur à poche

## FIG. 14

EP 2 861 278 B1

**FIG. 15**

**EP 2 861 278 B1**

## RÉFÉRENCES CITÉES DANS LA DESCRIPTION

### Documents brevets cités dans la description

- WO 9745150 A
- KR 20020036248 A